(19) Europäisches Patentamt
European Patent Office
Office européen des brevets

(11) Veröffentlichungsnummer: **0 025 936**
**A1**

(12) # EUROPÄISCHE PATENTANMELDUNG

(21) Anmeldenummer: 80105380.2

(22) Anmeldetag: 09.09.80

(51) Int. Cl.³: C 09 B 57/06
C 07 D 487/04

(30) Priorität: 22.09.79 DE 2938467

(43) Veröffentlichungstag der Anmeldung:
01.04.81 Patentblatt 81/13

(84) Benannte Vertragsstaaten:
AT BE CH DE FR GB IT LI LU NL SE

(71) Anmelder: BASF Aktiengesellschaft
Carl-Bosch-Strasse 38
D-6700 Ludwigshafen(DE)

(72) Erfinder: Kanter, Hartmut, Dr.
Kolpingstrasse 3B
D-6710 Frankenthal(DE)

(72) Erfinder: Schefczik, Ernst, Dr.
Dubliner Strasse 7
D-6700 Ludwigshafen(DE)

(54) **Naphtholactamderivate und deren Verwendung als Farbstoffe.**

(57) Die Erfindung betrifft Naphtholactamderivate der allgemeinen Formel I

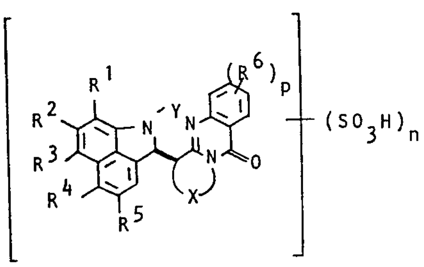

I

in der
n 0, 1, 2 oder 3,
p 0, 1, 2 oder 3,
$R^1$ Wasserstoff, Chlor, Brom, Alkyl, Alkoxy, $NO_2$ oder Arylmercapto,
$R^2$ Wasserstoff oder Chlor,
$R^3$ Wasserstoff, Chlor, Brom, Alkyl, Alkoxy, Nitro, Acylamino, Alkylsulfonylamin, Arylsulfonylamino, Alkylmercapto, Arylmercapto, Arylsulfonyl, Alkylsulfonyl, gegebenenfalls N-substituiertes Sulfamid, Alkanoyl, Aroyl, Alkoxycarbonyl, gegebenenfalls substituiertes Carbamoyl oder Cyan,
$R^4$ Wasserstoff, Chlor, Alkoxy, Arylmercapto, Alkylmercapto, Alkoxycarbonyl, gegebenenfalls substituiertes Carbamoyl oder Cyan,
$R^3$ und $R^4$ zusammen einen Rest der Formel

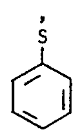 oder 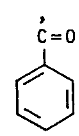 ,

$R^5$ Wasserstoff, Chlor oder Alkoxy,
$R^6$ Fluor, Chlor, Brom, Methyl, Trifluormethyl, Nitro, gegebenenfalls substituiertes Carbamoyl oder Sulfamoyl oder Phenylmercapto,
X einen Rest der Formel

-CONH-, -CONR-, -CSNH-, $-\underset{SR}{\underset{|}{C}}=N-$, $-\underset{C_1-C_8\text{-Alkyl}}{\underset{|}{C}}=N-$ oder

./...

EP 0 025 936 A1

-C=N- und
Aryl

Y Wasserstoff oder 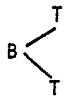 bedeuten, wobei

T Fluor, Chlor, Brom, gegebenenfalls substituiertes Aryl oder ein Acryloxyrest ist.

Die Verbindungen eignen sich zum Färben synthetischer Fasern und von Kunststoffen in der Masse.

BASF Aktiengesellschaft O. Z. 0050/034055

BEZEICHNUNG GEÄNDERT
siehe Titelseite

Naphtholactamderivate

Die Erfindung betrifft Verbindungen der Formel I

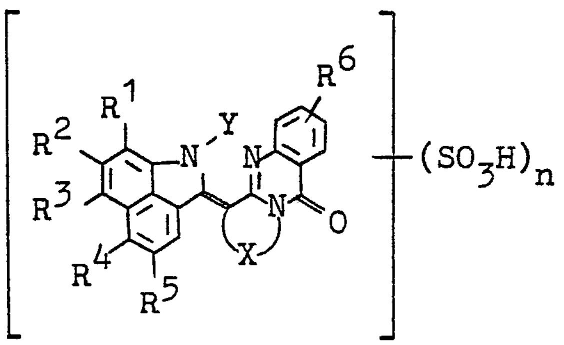

I

in der

n 0, 1, 2 oder 3,

p 0, 1, 2 oder 3

$R^1$ Wasserstoff, Chlor, Brom, Alkyl, Alkoxy, $NO_2$ oder Arylmercapto,

$R^2$ Wasserstoff oder Chlor,

$R^3$ Wasserstoff, Chlor, Brom, Alkyl, Alkoxy, Nitro, Acylamino, Alkylsulfonylamino, Arylsulfonylamino, Alkylmercapto, Arylmercapto, Arylsulfonyl, Alkylsulfonyl, gegebenenfalls N-substituiertes Sulfamid, Alkanoyl, Aroyl, Alkoxycarbonyl, gegebenenfalls substituiertes Carbamoyl oder Cyan,

$R^4$ Wasserstoff, Chlor, Alkoxy, Arylmercapto, Alkylmercapto, Alkoxycarbonyl, gegebenenfalls substitu-

Bg/BL

iertes Carbamoyl oder Cyan,

$R^3$ und $R^4$ zusammen einen Rest der Formel

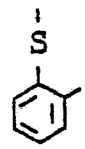 oder 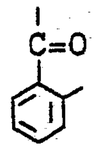 ,

$R^5$ Wasserstoff, Chlor oder Alkoxy,

$R^6$ Fluor, Chlor, Brom, Methyl, Trifluormethyl, Nitro, gegebenenfalls substituiertes Carbamoyl oder Sulfamoyl oder Phenylmercapto,

X einen Rest der Formel

-CONH-, -CONR-, -CSNH-, $-\underset{SR}{C}=N-$, $-\underset{C_1-C_8\text{-Alkyl}}{C}=N-$ oder $-\underset{Aryl}{C}=N-$ und

Y Wasserstoff oder $B{<}^{T}_{T}$ bedeuten, wobei

T Fluor, Chlor, Brom, gegebenenfalls substituiertes Aryl oder ein Acyloxyrest ist.

n ist vorzugsweise O.

Erläuterungen zu $R^1$ bis $R^5$:

Alkyl-, Alkoxy- und Arylmercaptogruppen für $R^1$ sind z. B. Methyl, Äthyl, Methoxy, Äthoxy, Phenylmercapto oder durch Chlor, Methyl, Methoxy, Phenoxy oder Methoxycarbonyl substituiertes Phenylmercapto.

Für $R^3$ sind die gleichen Reste wie für $R^1$ zu nennen sowie Naphthylmercapto, Benzthiazolyl-2-mercapto, Acylaminophenylmercapto, Diacyliminophenylmercapto, Acetylamino, Propionylamino, Benzoylamino, durch Chlor, Methyl oder Methoxy substituiertes Benzoylamino, Methylsulfonylamino, Äthylsulfonylamino, Phenylsulfonylamino,

Tolylsulfonylamino, Methylmercapto, Äthylmercapto, ß-Hydroxyäthylmercapto, ß-Hydroxypropylmercapto, Butylmercapto, Methylsulfonyl, Äthylsulfonyl, Phenylsulfonyl, Tolylsulfonyl, Chlorphenylsulfonyl, Acetyl, Chloracetyl, Propionyl, Butyryl, Benzoyl, durch Methyl, Methoxy, Chlor oder Brom substituiertes Benzoyl, Sulfamoyl, N-Methyl-, N-Äthyl-, N-Butyl-, N-Phenyl-, N-Chlorphenyl-, N-Methylphenyl-, N-Methoxyphenyl-, N-Trifluormethylphenyl-, N-Methyl-N-phenyl-, N,N-Dimethyl-, N,N-Diäthyl-, N,N-Dipropyl- oder N,N-Dibutylsulfamoyl, Pyrrolidinosulfonyl, Piperidinosulfonyl oder Morpholinosulfonyl.

Ferner kommen die Reste der Formeln $NHCONH_2$, $NHCONHCH_3$ oder $NHCONHC_6H_5$ in Betracht.

Reste $R^4$ sind neben Wasserstoff und Chlor die für $R^1$ genannten Alkoxy- und Arylmercaptoreste.

Alkoxycarbonylreste $R^3$ und $R^4$ sind z. B. Methoxycarbonyl, Äthoxycarbonyl, n- und iso-Propoxycarbonyl, Butoxycarbonyl, 2-Äthylhexoxycarbonyl, ß-Hydroxyäthoxycarbonyl, ß-Alkoxyäthoxycarbonyl oder ß-Dimethylaminoäthoxycarbonyl.

Als substituierte Carbamoylreste seien beispielsweise genannt: Methylaminocarbonyl, Äthyl-, Propyl-, iso-Propyl-, Butyl-, iso-Butyl- und 2-Äthylhexylaminocarbonyl, ß-Hydroxyäthylaminocarbonyl, ß-Methoxyäthylaminocarbonyl, γ-Hydroxy-, γ-Methoxy-, γ-Äthoxy, γ-Butoxypropylaminocarbonyl, Dimethyl-, Diäthyl-, Dibutylaminocarbonyl, Pyrrolidinocarbonyl, Piperidinocarbonyl und Morpholinocarbonyl. Diese Reste sowie die entsprechen-

den Sulfamoylreste kommen auch für $R^6$ in Betracht.

Alkoxyreste $R^5$ sind z.B. Methoxy oder Äthoxy.

Gegebenenfalls substituierte Arylreste T sind z.B. durch Methyl, Äthoxy, Methoxy oder Äthoxy substituiertes Phenyl, bevorzugt ist unsubstituiertes Phenyl.

Zur Herstellung der Verbindungen der Formel I kann man Verbindungen der Formeln II

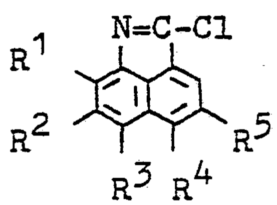 , 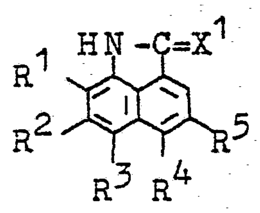 oder 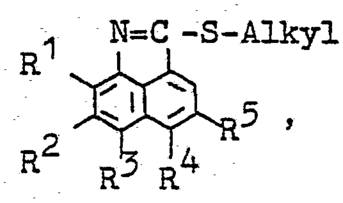 ,

wobei $X^1$ Sauerstoff oder Schwefel bedeutet und Alkyl 1 bis 4 C-Atome hat, mit Verbindungen der Formel III

III 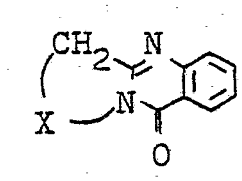

umsetzen, $R^1$ bis $R^6$ und X haben dabei die angegebenen Bedeutungen.

Für $X^1$ = O ist die Gegenwart eines Kondensationsmittels notwendig, für die anderen Verbindungen II ist dies nicht erforderlich.

Als Kondensationsmittel eignen sich Phosphorhalogenide wie Phosphorpentachlorid, Phosphortrichlorid oder Phosphoroxytribromid und insbesondere Phosphoroxytrichlorid.

Man kann die Umsetzung in einem inerten Lösungsmittel, wie Glykol- und Polyglykoldiäthern, Butyrolacton, Toluol, Chlorbenzol, Dichlorbenzol, Nitrobenzol oder Dioxan oder auch in einem Überschuß des Kondensationsmittels vornehmen.

Bei der Umsetzung der Naphtholactamderivate ohne Kondensationsmittel eignen sich ebenfalls die schon genannten Lösungsmittel, zusätzlich sind z. B. Pyridin, Eisessig, Dimethylformamid oder N-Methylpyrrolidon zu nennen.

Einzelheiten der Reaktionsführung und -bedingungen können den Beispielen entnommen werden, in denen sich Angaben über Teile und Prozente, sofern nicht anders vermerkt, auf das Gewicht beziehen.

Die Verbindungen der Formel I mit Y = $-B{<}^{T}_{T}$ können auch in einer tautomeren Form der Formel

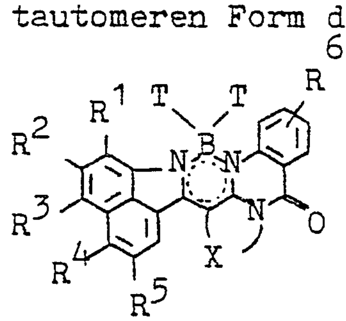

existieren. Man erhält sie aus den Verbindungen mit Y = H durch Umsetzung mit Borsäurederivaten, wie Halogeniden. Man auch Borsäure oder eine Arylborsäure in Gegenwart von Carbonsäureanhydriden umsetzen und erhält dann die Acyloxyderivate mit T = Acyloxy. Einzelheiten der Herstellung können ebenfalls den Beispielen entnommen werden.

Von besonderer Bedeutung sind Verbindungen der Formel I a

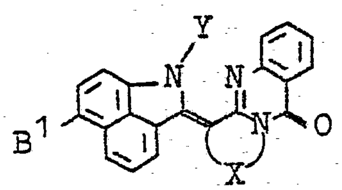

I a

in der

$B^1$ Wasserstoff, Halogen, Aroyl oder Arylmercapto,

X einen Rest der Formel -CONR- oder $-\underset{CH_3}{\underset{|}{C}}=N-$ und

Y Wasserstoff, $BF_2$, $B(Aryl)_2$, $B\begin{matrix}\diagup Aryl \\ \diagdown OCOC_1\text{- bis }C_4\text{-Alkyl}\end{matrix}$ oder B ($OCOC_1$- bis $C_4$-Alkyl$)_2$

bedeuten.

Die Verbindungen der Formel I sind orange bis blau und eignen sich als Dispersionsfarbstoffe für synthetische Fasern, insbesondere Polyester sowie zum Färben von Kunststoffen in der Masse. Als Kunststoffe seien z. B. Polystyrol, Polymethacrylat, Polycarbonat, Polyurethane, Polyamide oder Polyolefine genannt. Die Färbungen zeichnen sich durch Brillanz und gute Echtheiten aus. Die Borverbindungen zeigen eine starke Fluoreszenz und die Verbindungen mit n = 1, 2 oder 3 eignen sich zum Färben von natürlichen und synthetischen Polyamiden.

Beispiel 1

In 1500 Raumteile Dichlorbenzol werden 277 Teile 4-Phenylmercaptonaphtholactam und 200 Teile der Verbindung

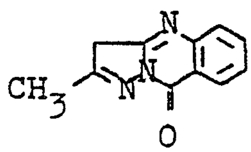

eingetragen und bei 90 °C gerührt. Dazu tropft man innerhalb einer Stunde 195 Teile Phosphoroxitrichlorid. Man rührt weitere 6 Stunden bei 100 °C, senkt die Temperatur auf 70 °C und tropft ein Gemisch aus 500 Raumteilen Methanol und 150 Teilen Triäthylamin zu. Nach dem Erkalten wird abgesaugt, mit Methanol gewaschen und getrocknet. Man erhält 369 Teile des Farbstoffs

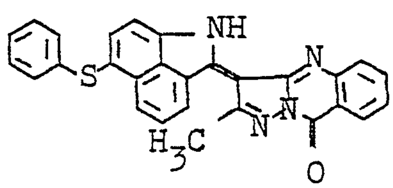

in Form eines dunklen Kristallpulvers. Der Farbstoff löst sich in Dimethylformamid mit blauvioletter Farbe und gibt auf Fasern und Geweben aus Polyester aus wäßrigem Bad rotstichige Blaufärbungen mit guter Licht- und Thermofixierechtheit.

Beispiel 2

169 Teile Naphtholactam und 220 Teile der Verbindung

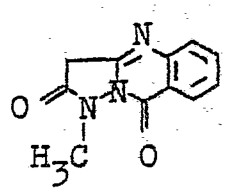

werden in 1000 Raumteile Chlorbenzol eingetragen und bei 80 °C gerührt. Dazu tropft man 200 Teile Phosphoroxitrichlorid und rührt 4 Stunden bei 90 °C nach. Nun werden, bei abgestellter Heizung, 400 Teile Methanol nach und nach zugegeben, wobei das Reaktionsgemisch ins Sieden kommt und Chlorwasserstoff entweicht. Nach dem Erkalten wird abgesaugt, mit Methanol und mit verd. wäßrigem Ammoniak gewaschen und getrocknet. Man erhält 274 Teile des Farbstoffs

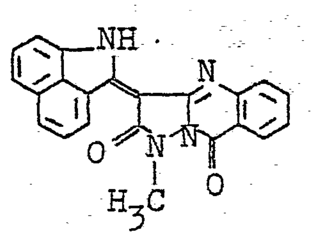
,

der Polyester aus wäßrigem Bad in brillanten Scharlachtönen von sehr guter Licht- und Thermofixierechtheit anfärbt. Beim Massefärben von Thermoplasten erhält man mit dem Farbstoff brillante Rotorangefärbungen mit ausgezeichneter Lichtechtheit. Farbstoffe mit etwas röteren Farbtönen erhält man, wenn man die substituierten Verbindungen

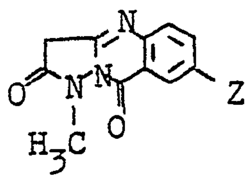

Z = Cl, Br, $-SO_2N(C_4H_9)_2$,
$-SO_2OC_6H_5$

mit Naphtholactam, wie beschrieben, kondensiert.

Beispiel 3

In 1200 Raumteile Pyridin werden 327 Teile

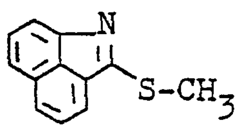 · HJ

und 245 Teile

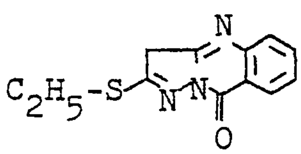

eingetragen und 8 Stunden unter Rückfluß gekocht. Der Kristallbrei wird mit 400 Teilen Methanol verdünnt und nach dem Erkalten abgesaugt. Man erhält nach dem Trocknen 313 Teile des Farbstoffs

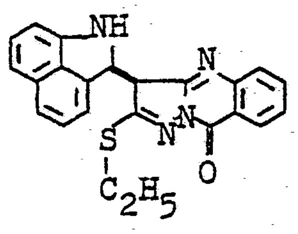

,

der Fasern und Gewebe aus Polyester aus wäßrigem Bad in brillanten Rottönen von sehr guter Lichtechtheit anfärbt. Farbstoffe mit praktisch gleichem Farbton erhält man, wenn man die Verbindungen

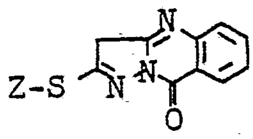

Z = $-CH_2CH_2OH$, $-CH_2C_6H_5$

anstelle der S-Äthylverbindung einsetzt.

Beispiel 4

In 1200 Raumteile Dimethylformamid werden 224 Teile der Verbindung

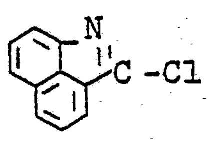

· HCl

und 270 Teile der Verbindung

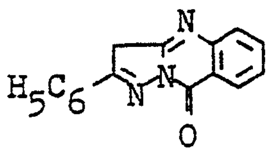

eingetragen und 4 Stunden bei 95 °C gerührt. Dann stellt man die Heizung ab, gibt erst 800 Raumteile Methanol, dann 100 Teile wasserfreies Natriumacetat zu und rührt 30 Minuten bei 65 °C. Nun wird erkalten gelassen, abgesaugt, mit Methanol und mit warmem Wasser gewaschen und getrocknet. Man erhält 371 Teile des Farbstoffs

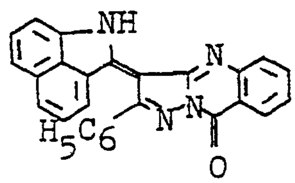

in Form eines dunklen Kristallpulvers. Mit dem Farbstoff werden auf Polyestergewebe brillante violette Drucke mit sehr guten Echtheiten erhalten.

Farbstoffe mit sehr ähnlichem Farbton werden erhalten, wenn man anstelle des Phenylpyrazolochinazolons äquivalente Mengen der substituierten Verbindungen

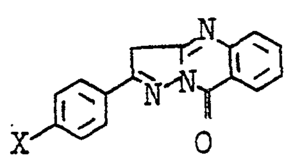 X = $-CH_3$, $-OCH_3$, $-Cl$

einsetzt.

Nach den in den Beispielen 1 bis 4 angegebenen Verfahren wurden aus den Ausgangsstoffen die folgenden Farbstoffe hergestellt:

| Bsp. | | | | Farbton |
|---|---|---|---|---|
| 5 | 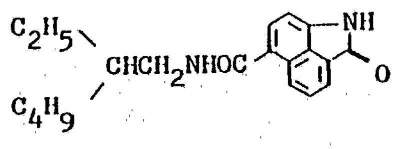 | 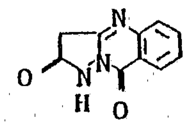 | 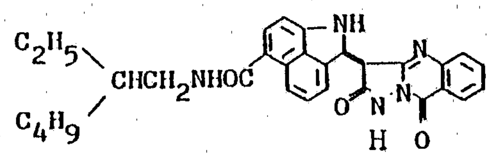 | rot |
| 6 | 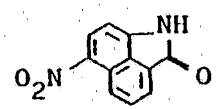 | 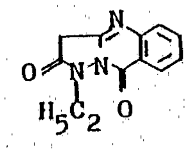 | 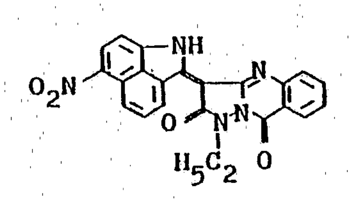 | blaustichig rot |
| 7 | 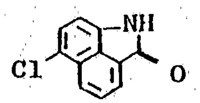 | " | 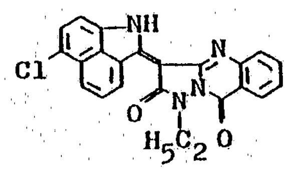 | rot |
| 8 | 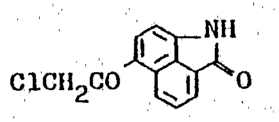 | " | 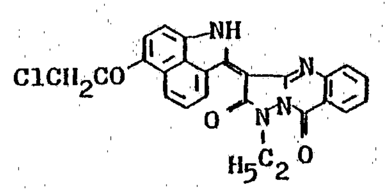 | rot |

| Bsp. | | | | Farbton |
|---|---|---|---|---|
| 9 | 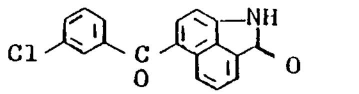 | 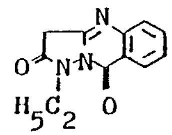 | 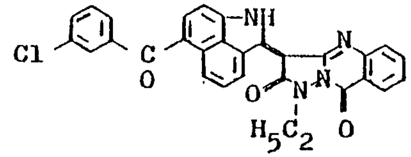 | rot |
| 10 | 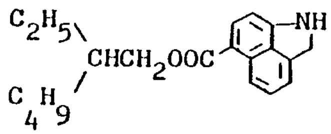 | " | 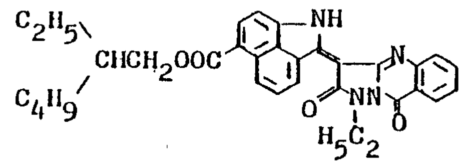 | rot |
| 11 | 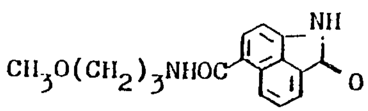 | " | 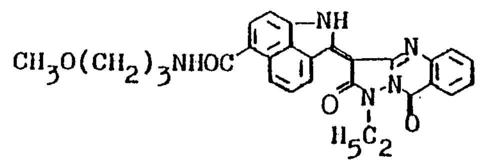 | rot |
| 12 | 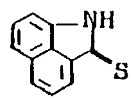 | 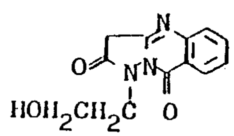 | 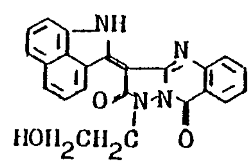 | rotorange |

| Bsp. | | | | Farbton |
|---|---|---|---|---|
| 13 | 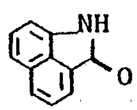 | 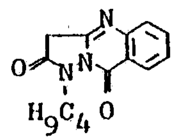 | 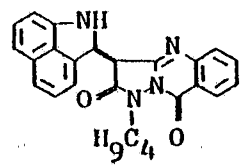 | rotorange |
| 14 | 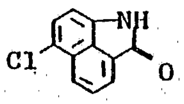 | " | 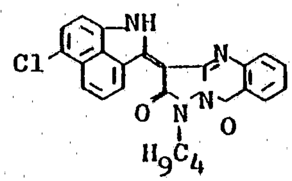 | rot |
| 15 | 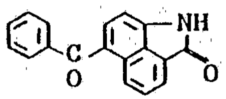 | " | 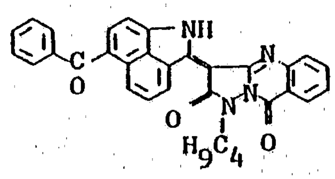 | rot |
| 16 | 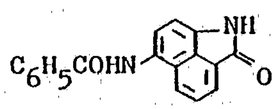 | " | 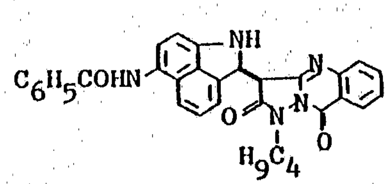 | rotviolett |

| Bsp. | | | | Farbton |
|---|---|---|---|---|
| 17 | 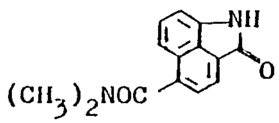 | 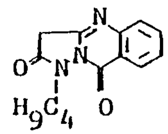 | 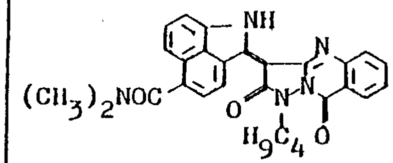 | rot |
| 18 | 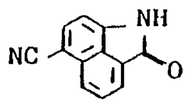 | " | 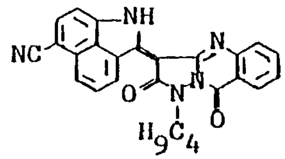 | blaustichig rot |
| 19 | 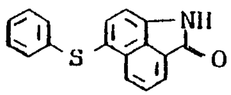 | " | 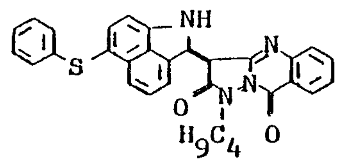 | rotviolett |
| 20 | 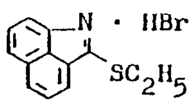 | 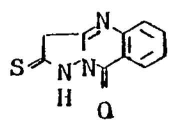 | 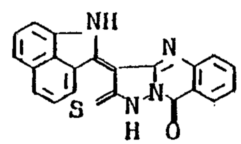 | rot |

| Bsp. | | | | Farbton |
|---|---|---|---|---|
| 21 | 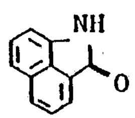 | 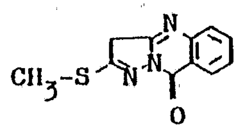 | 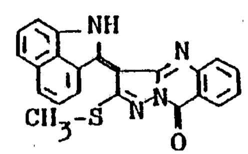 | rot |
| 22 | 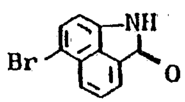 | " | 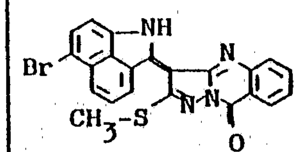 | rot |
| 23 | 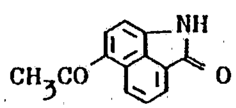 | " | 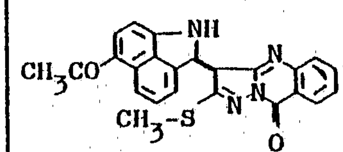 | rot |
| 24 | 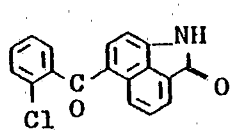 | 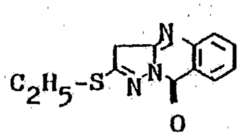 | 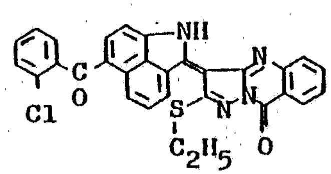 | rot |

| Bsp. | | | | Farbton |
|---|---|---|---|---|
| 25 | 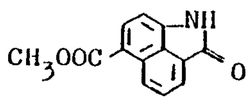 | 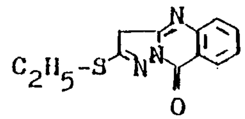 | 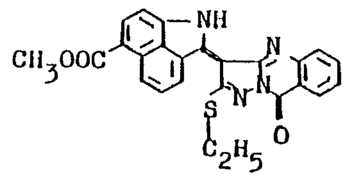 | rot |
| 26 | 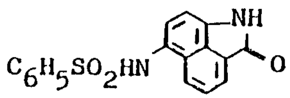 | " | 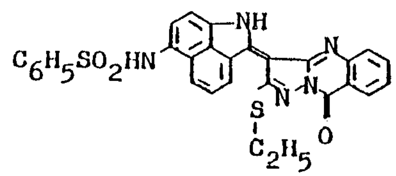 | rotviolett |
| 27 | 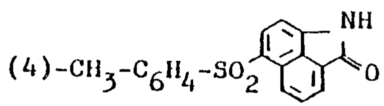 | " | 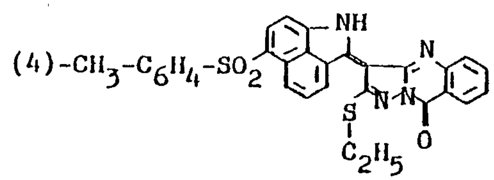 | rot |
| 28 | 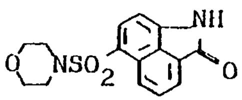 | " | 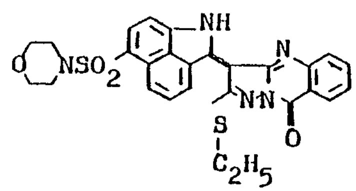 | rot |

| Bsp. | | | Farbton |
|---|---|---|---|
| 29 | 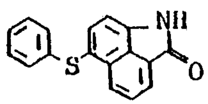 | 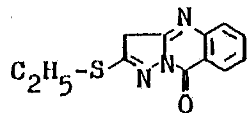 | 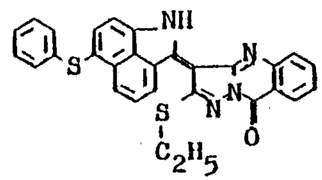 | rotviolett |
| 30 | 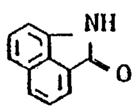 | 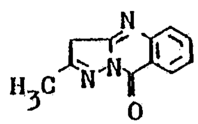 | 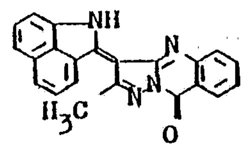 | violett |
| 31 | 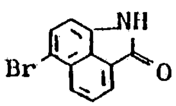 | " | 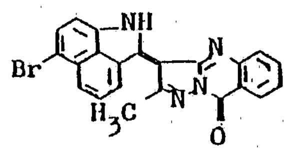 | " |
| 32 | 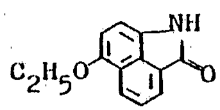 | " | 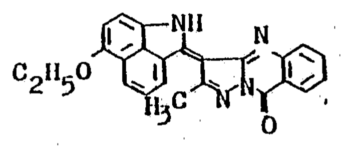 | " |

| Bsp. | | | | Farbton |
|---|---|---|---|---|
| 33 | 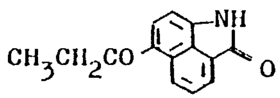 | 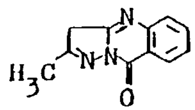 | 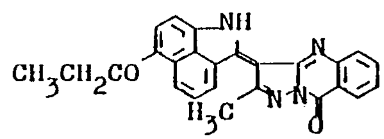 | violett |
| 34 | 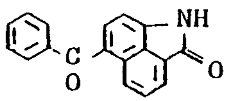 | " | 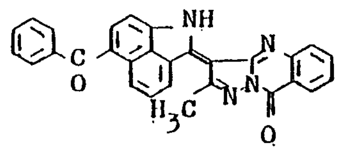 | " |
| 35 | 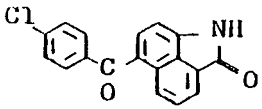 | " | 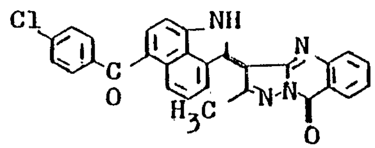 | " |
| 36 | 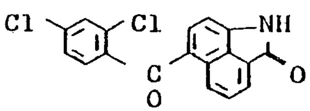 | " | 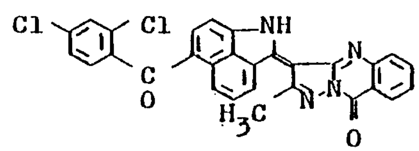 | " |

| Bsp. | | | | Farbton |
|---|---|---|---|---|
| 37 | 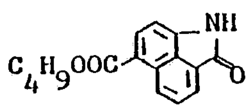 | 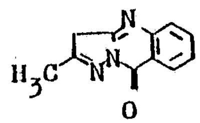 | 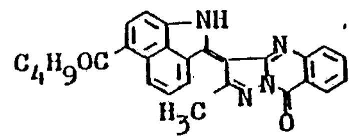 | violett |
| 38 | 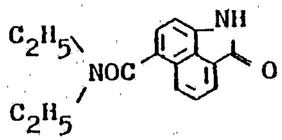 | " | 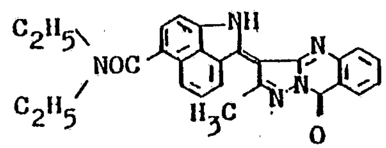 | " |
| 39 | 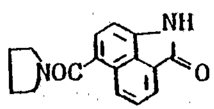 | " | 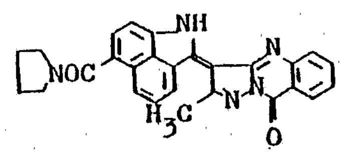 | " |
| 40 | 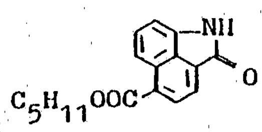 | " | 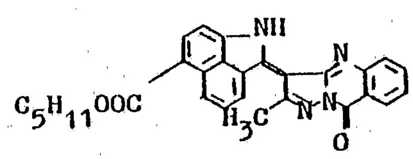 | " |

| Bsp. | | | | Farbton |
|---|---|---|---|---|
| 41 | 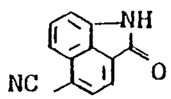 | 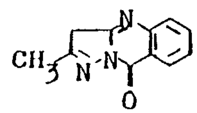 | 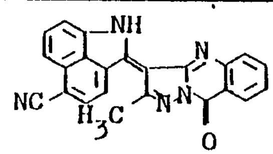 | violett |
| 42 | 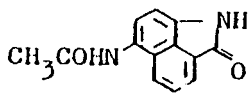 | " | 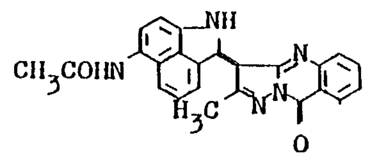 | blauviolett |
| 43 | 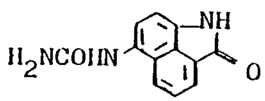 | " | 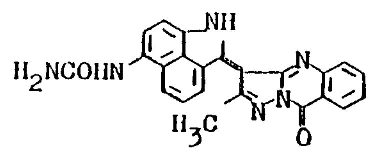 | " |
| 44 | 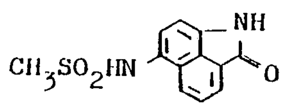 | " | 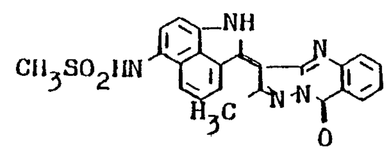 | " |

| Bsp. | | | | Farbton |
|---|---|---|---|---|
| 44 | 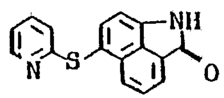 | 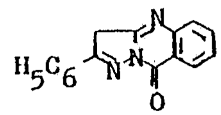 | 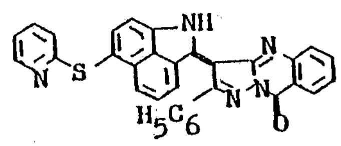 | blauviolett |
| 45 | 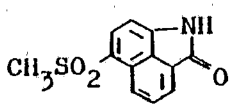 | 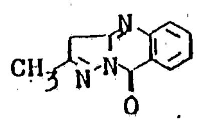 | 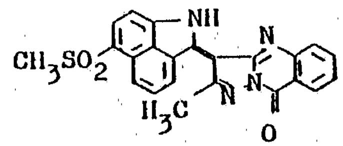 | violett |
| 46 | 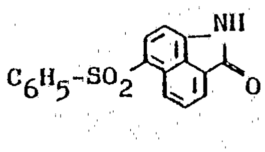 | " | 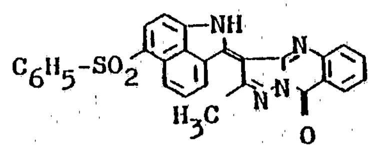 | " |
| 47 | 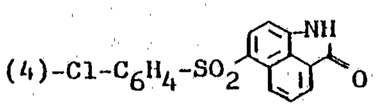 | " | 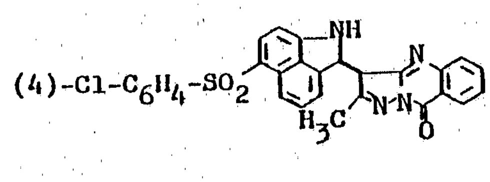 | " |

| Bsp. | | | | Farbton |
|---|---|---|---|---|
| 48 | 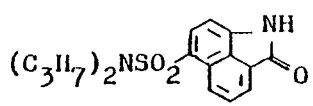 | 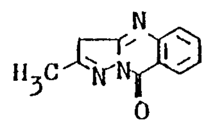 | 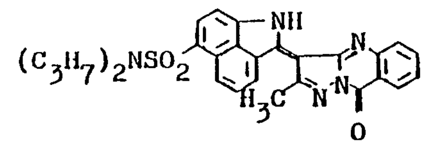 | violett |
| 49 | 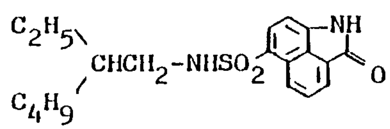 | " | 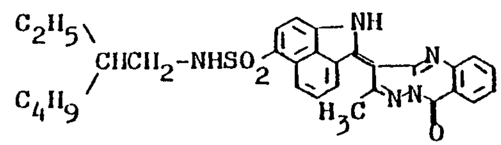 | " |
| 50 | 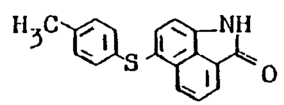 | " | 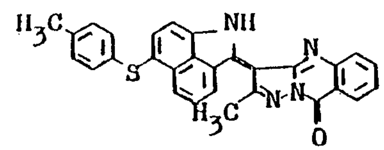 | rotstichig blau |
| 51 | 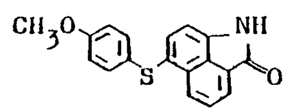 | " | 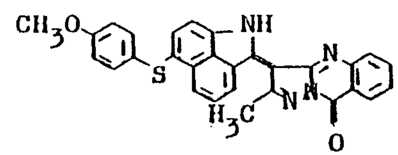 | " |

| Bsp. | | | | Farbton |
|---|---|---|---|---|
| 52 | 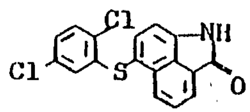 | 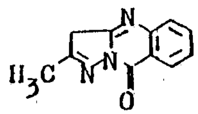 | 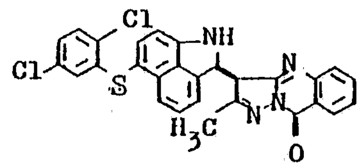 | rotstichig blau |
| 53 | 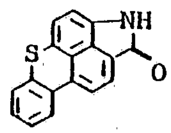 | " | 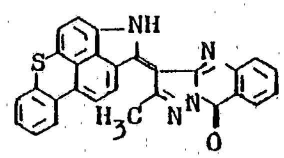 | blau |
| 54 | 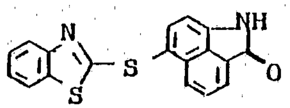 | " | 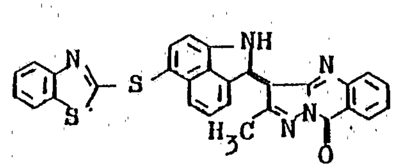 | violett |
| 55 | 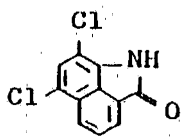 | " | 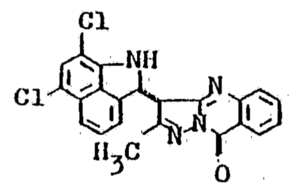 | " |

| Bsp. | | | | Farbton |
|---|---|---|---|---|
| 56 | 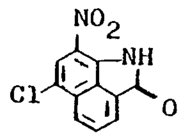 | 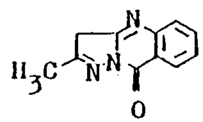 | 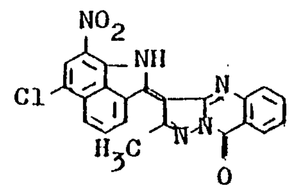 | violett |
| 57 | 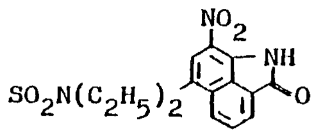 | " | 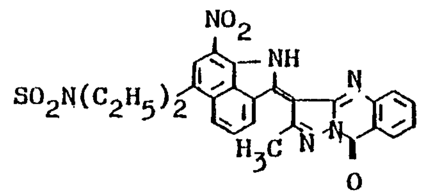 | " |
| 58 | 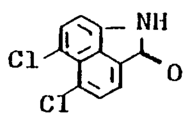 | " | 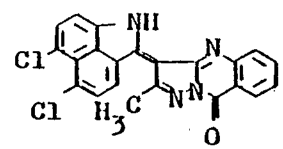 | " |
| 59 | 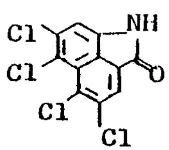 | " | 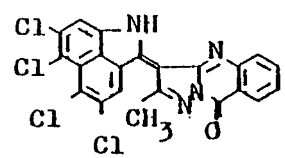 | " |

| Bsp. | | | | Farbton |
|---|---|---|---|---|
| 60 | 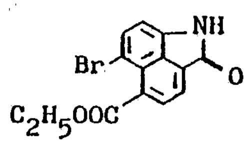 | 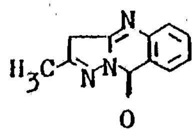 | 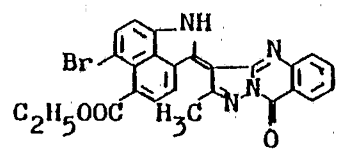 | violett |
| 61 | 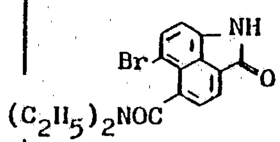 | " | 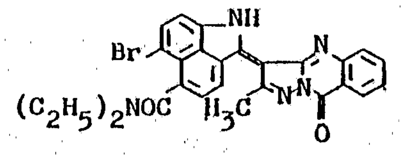 | " |
| 62 | 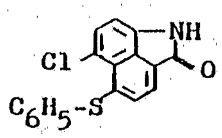 | " | 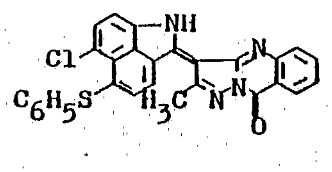 | blauviolett |
| 63 | 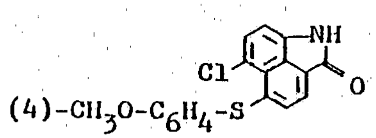 | " | 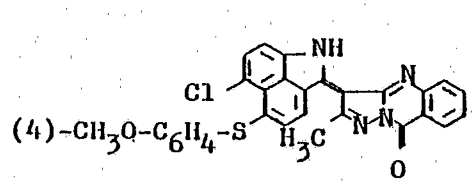 | " |

| Bsp. | | | | Farbton |
|---|---|---|---|---|
| 64 | 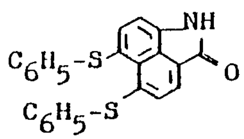 | 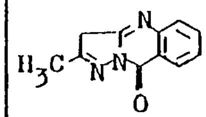 | 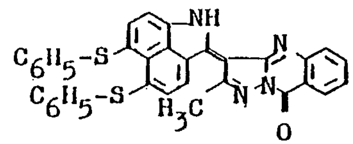 | blau |
| 65 | 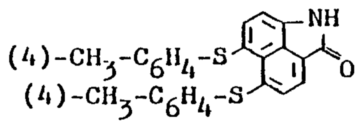 | " | 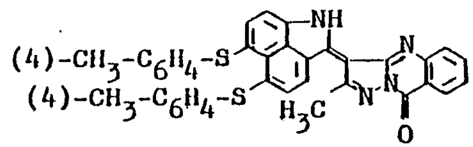 | " |
| 66 | 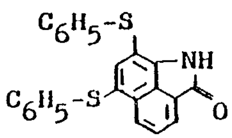 | " | 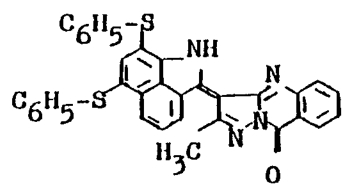 | " |
| 67 | 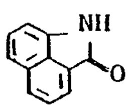 | 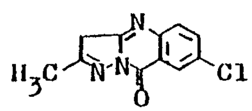 | 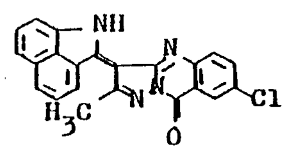 | violett |

| Bsp. | | | | Farbton |
|---|---|---|---|---|
| 68 | 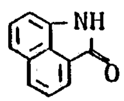 | 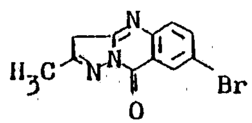 | 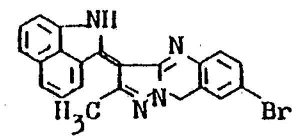 | violett |
| 69 | " | 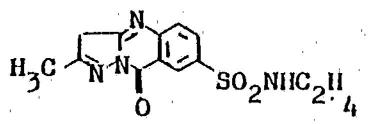 | 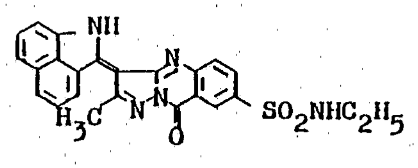 | " |
| 70 | " | 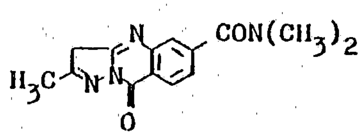 | 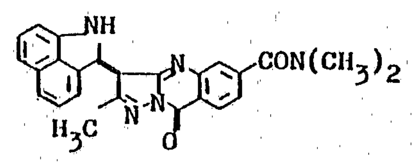 | " |
| 71 | " | 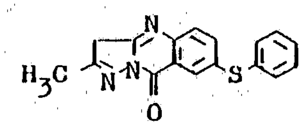 | 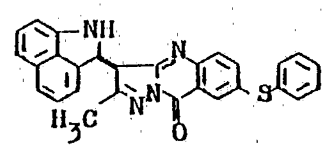 | " |

| Bsp. | | | | Farbton |
|---|---|---|---|---|
| 72 | 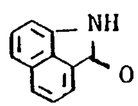 | 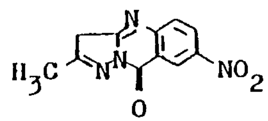 | 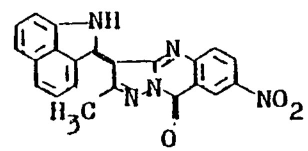 | violett |
| 73 | " | 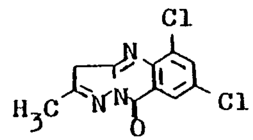 | 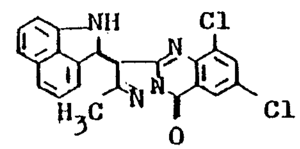 | " |
| 74 | " | 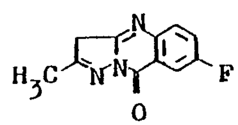 | 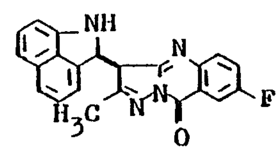 | " |
| 75 | " | 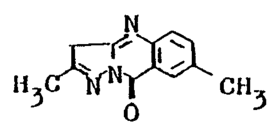 | 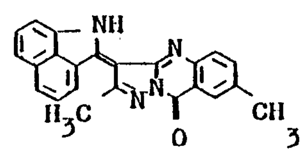 | " |

| Bsp. | | | Farbton |
|---|---|---|---|
| 76 | 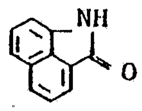 | 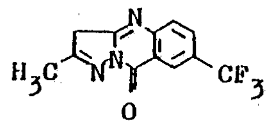 | 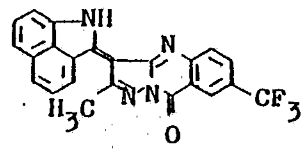 | violett |
| 77 | 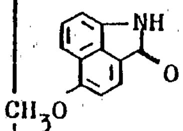 | 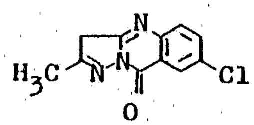 | 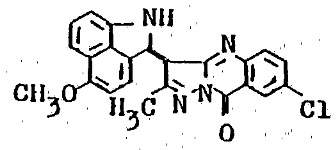 | " |
| 78 | 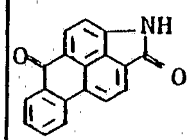 | 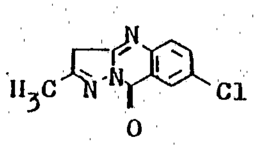 | 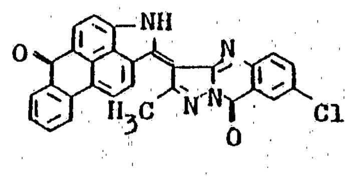 | blauviolett |
| 79 | 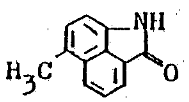 | 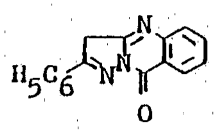 | 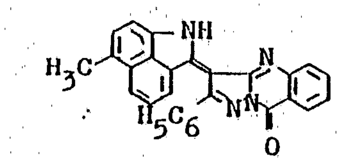 | violett |

| Bsp. | | | | Farbton |
|---|---|---|---|---|
| 80 | 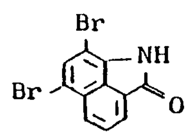 | 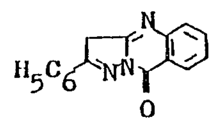 | 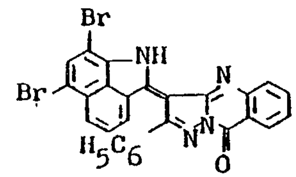 | violett |
| 81 | 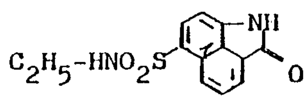 | " | 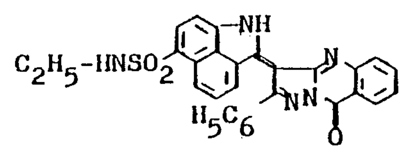 | " |
| 82 | 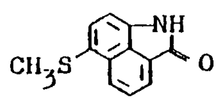 | " | 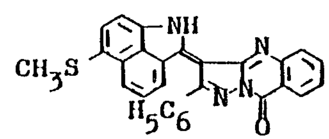 | blauviolett |
| 83 | 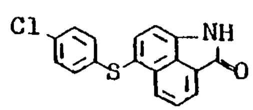 | " | 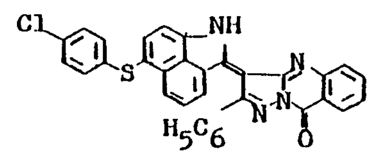 | rotstichig blau |

Beispiel 84

In 90 Teile rauchende Schwefelsäure mit einem Schwefeltrioxidgehalt von 8 % werden 10,5 Teile des nach Bsp. 30 erhaltenen Farbstoffs eingetragen und 12 Stunden bei Raumtemperatur gerührt. Dann wird der Ansatz auf Eis gegossen, die ausgefallene Sulfosäure abgesaugt und mit kaltem Wasser gewaschen. Man erhält nach dem Trocknen 12,1 Teile der Verbindung

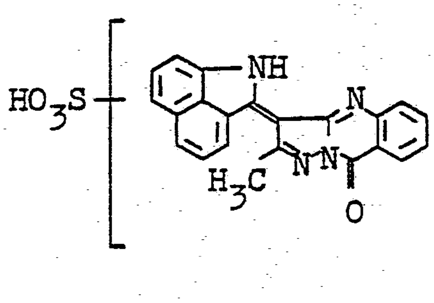

die sich in heißem Wasser mit blaustichig roter Farbe, in Alkalien mit roter Farbe löst. Der Farbstoff gibt beim Färben auf Gewebe aus natürlichen oder synthetischen Polyamiden aus wäßrigem Bad brillante Violettfärbungen mit guter Lichtechtheit.

Beispiel 85

In 1500 Raumteile entwässertes Chlorbenzol werden 267,5 Teile Naphtholactam-4-sulfochlorid und 200 Teile der Verbindung

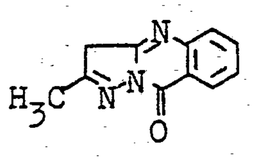

eingetragen und bei 90 °C gerührt. Dazu tropft man 225 Teile Phosphoroxitrichlorid und rührt weitere 5 Stunden bei 90 - 95 °C. Nun wird überschüssiges Phosphortrichlorid durch vorsichtige Zugabe von 100 Raumteilen Ethanol zersetzt und das Chlorbenzol mit Wasserdampf abdestilliert. Die wäßrige Farbstoffsuspension wird ammoniakalisch gestellt, heiß filtriert und das Filtrat in der Kälte mit conc. Salzsäure angesäuert. Die ausgefallene Sulfosäure wird abgesaugt, mit Eiswasser gewaschen und getrocknet. Man erhält 372 Teile des Farbstoffs

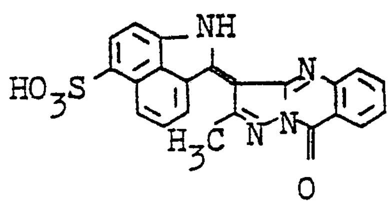

der die gleichen coloristischen Eigenschaften wie der Farbstoff des Beispiel 84 hat.

Beispiel 86

In 70 Teile rauchende Schwefelsäure mit 23 % freiem Schwefeltrioxid werden 10 Teile des nach Beispiel 67 erhaltenen Farbstoffs eingetragen und 6 Stunden bei 35 °C gerührt. Nach Aufarbeitung, wie im Beispiel 84 beschrieben, erhält man 11,3 Teile der Verbindung

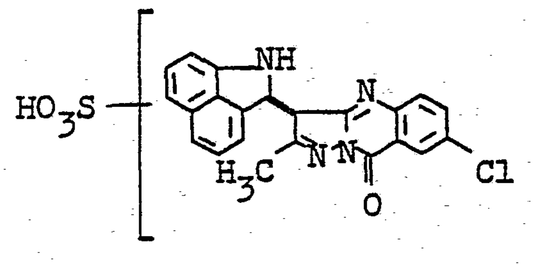

mit einem Chlorgehalt von 7,4 % (berechnet 7,7 %). Mit dem Farbstoff werden auf Geweben aus synthetischen Polyamiden brillante Violettfärbungen erhalten.

Nach dem gleichen Verfahren lassen sich auch die Farbstoffe der Beispiele 31, 34, 45, 46, 55, 58, 68, 73 und 75 in Sulfosäuren überführen, die alle aus wäßrigem Bad Violettfärbungen auf Polyamidmaterial geben.

Beispiel 87

10 Teile des nach Beispiel 2 erhaltenen Farbstoffs werden in 75 Teile rauchende Schwefelsäure mit einem Gehalt von 23 % freiem Schwefeltrioxid eingetragen und 16 Stunden bei 20 - 25 °C gerührt. Nach Aufarbeitung analog Beispiel 84 erhält man 11,7 Teile der Verbindung

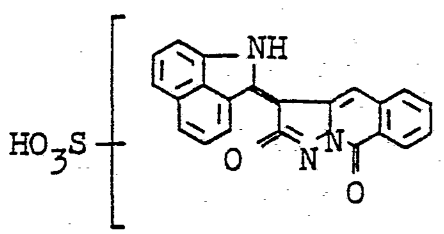

die sich in heißem Wasser mit roter Farbe löst. Der Farbstoff liefert auf Geweben aus natürlichen und synthetischen Polyamiden aus wäßrigem Bad brillante Rotfärbungen mit guter Lichtechtheit.

Beispiel 88

10 Teile des nach Beispiel 4 erhaltenen Farbstoffs werden in 60 Teile rauchende Schwefelsäure mit 60 % freiem Schwefeltrioxid eingetragen und 20 Stunden bei 25 - 30 °C gerührt. Dann gießt man das Reaktionsgemisch auf Eis/Natriumchlorid, saugt ab und wäscht mit 10 %iger Natriumchloridlösung nach. Man erhält einen natriumchloridhaltigen Preßkuchen des Farbstoffs

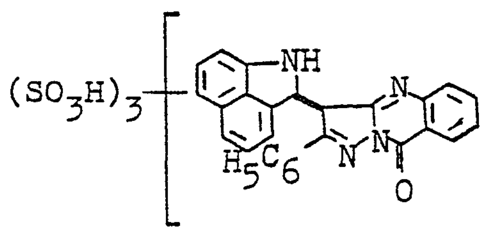

der auf Gewebe aus synthetischen Polyamiden brillante Violettfärbungen mit guter Lichtechtheit liefert.

Beispiel 89

In 100 Raumteile entwässertes Nitrobenzol werden 35 Teile des nach Beispiel 30 erhaltenen Farbstoffs und 12,5 Teile Borsäure eingetragen und bei 140 °C gerührt. Dazu tropft man innerhalb einer Stunde 100 Teile Essigsäureanhydrid und rührt 8 Stunden bei 140 °C. Nach Erkalten wird das Reaktionsprodukt abgesaugt und erst mit Essigsäure, dann mit Methanol gewaschen. Man erhält nach dem Trocknen 45,6 Teile der Verbindung

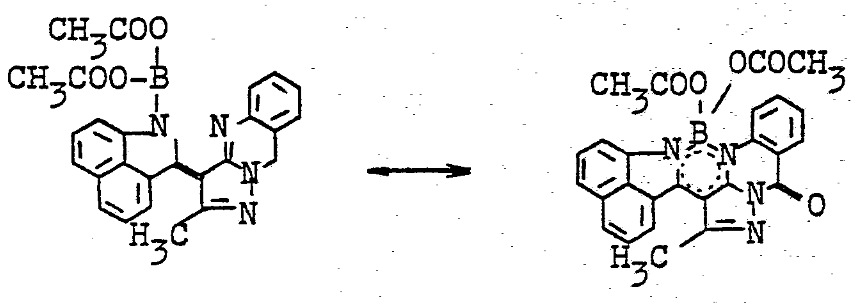

in Form metallglänzender Kristalle. Der Farbstoff ergibt beim Einarbeiten in Thermoplasten stark fluoreszierende gelbstichige Rotfärbungen mit ausgezeichneter Lichtechtheit.

Ersetzt man den Ausgangsstoff 30 durch äquivalente Mengen der Verbindungen 31, 32, 34, 35, 36, 37, 38, 40, 45, 46, 47, 48, 55, 58 oder 59 und verfährt sonst wie in Beispiel 90 beschrieben, so erhält man Farbstoffe mit sehr ähnlichem Farbton und coloristischen Eigenschaften.

Beispiel 90

Ein Gemisch aus 90 Raumteilen Dichlorbenzol, 125 Teilen Propionsäureanhydrid und 56,6 Teilen des nach Beispiel 64 erhaltenen Farbstoffs wird bei 145 °C gerührt und innerhalb einer Stunde anteilweise mit 12 Teilen Borsäure versetzt. Dann rührt man weitere 8 Stunden bei

145 - 150 °C, läßt erkalten und arbeitet, wie im Beispiel 89 beschrieben, auf. Man erhält 61,2 Teile der Verbindung

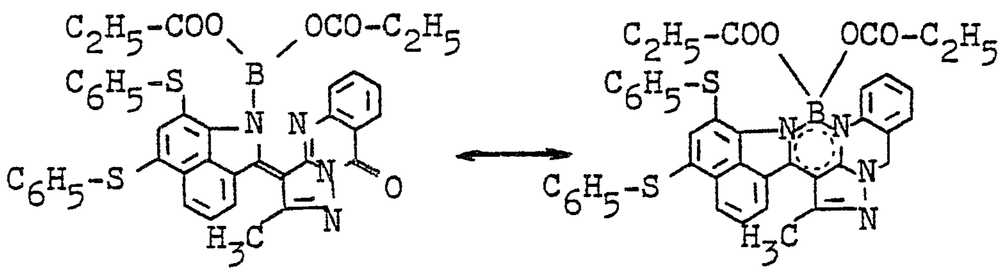

Der Farbstoff verleiht thermoplastischen Kunststoffen lichtechte, blaustichig rote Färbungen mit roter Fluoreszenz.

Farbstoffe mit praktisch gleichen coloristischen Eigenschaften werden erhalten, wenn man das Propionsäureanhydrid im Beispiel 90 durch 150 Teile Buttersäure- oder Isobuttersäureanhydrid ersetzt.

Beispiel 91

41,2 Teile des nach Beispiel 4 erhaltenen Farbstoffs werden in ein Gemisch aus 150 Raumteilen Dichlorbenzol und 100 Raumteilen Bortrifluorid-diätherat eingetragen und 6 Stunden unter Rückfluß gekocht. Nach dem Erkalten wird abgesaugt, mit Methanol gewaschen und getrocknet. Man erhält 39,3 Teile der Verbindung

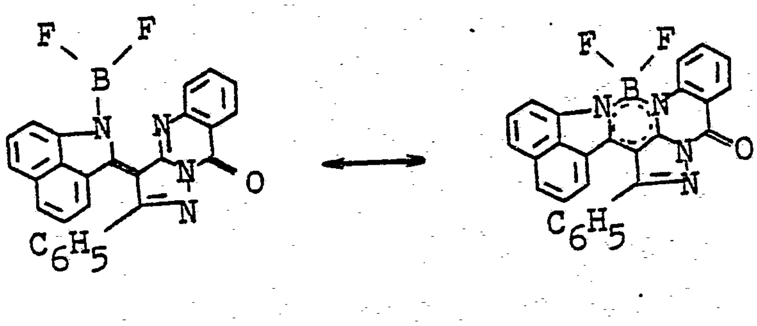

Die Verbindung gibt beim Einarbeiten in Thermoplasten wie z.B. Polystyrol oder Polymethacrylat fluoreszierende Rotfärbungen mit ausgezeichneten Echtheiten.

Beispiel 92

In 200 Raumteile entwässertes Chlorbenzol werden 16,9 Teile Naphtholactam und 23,3 Teile der Verbindung der Formel

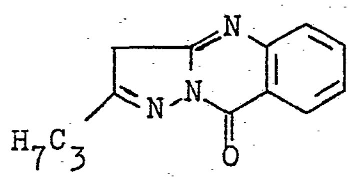

eingegeben und bei 100°C gerührt. Dazu tropft man 20 Teile Phosphoroxitrichlorid und rührt 5 Stunden bei 105°C. Dann werden 20 Raumteile Methanol zugetropft, das Reaktionsgemisch wird kurz aufgekocht und unter Rühren erkalten gelassen. Die abgeschiedenen Kristalle werden abgesaugt und mit verdünnter wäßriger Ammoniaklösung digeriert. Nach erneutem Absaugen, Waschen mit Wasser und Trocknen erhält man 35,2 Teile des Farbstoffs der Formel

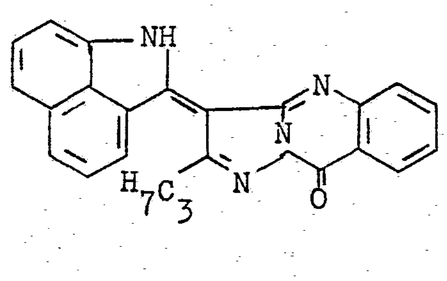

in Form dunkler Kristalle. Mit dem Farbstoff erhält man auf Fasern und Geweben aus Polyester kräftige Violettfärbungen mit guter Lichtechtheit und thermischer Beständigkeit.

Farbstoffe mit sehr ähnlichen coloristischen Eigenschaften werden erhalten, wenn man statt der Pyrazolochinazolonkomponente mit n-Propylrest die homologen Verbindungen

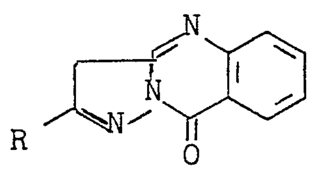

R = $-C_2H_5$, $-CH(CH_3)_2$, $-C_4H_9$, $-C_5H_{11}$ oder $-CH_2CHC_4H_9$
$\quad C_2H_5$

verwendet.

Beispiel 93

Zu einem Gemisch aus 100 Raumteilen Nitrobenzol, 12,8 Teilen Phenylborsäure $C_6H_5$ $B(OH_2)$ und 35,0 Teilen des nach Beispiel 30 erhaltenen Farbstoffs werden bei 140°C 100 Teile Essigsäureanhydrid getropft. Man kocht 6 Stunden unter Rückfluß ( ~ 145°C) und läßt unter Rühren erkalten. Dann werden die roten Kristalle abgesaugt, mit Nitrobenzol, Essigsäure und Aceton gewaschen und getrocknet; Ausbeute 44,0 Teile der Verbindung der Formel

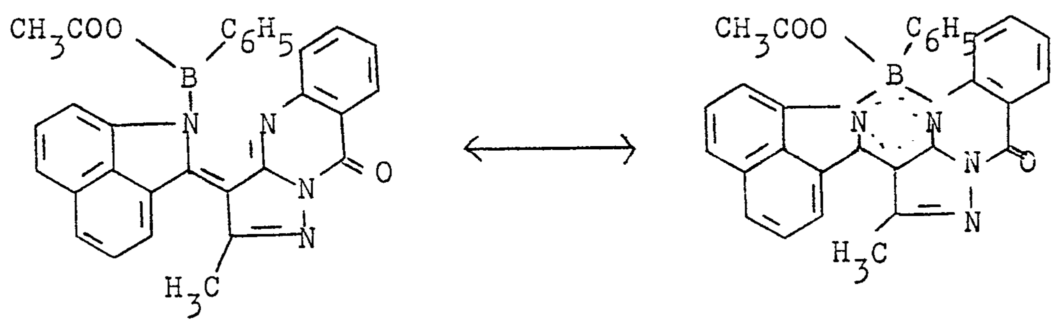

Beim Einarbeiten in Thermoplasten erhält man mit dem Farbstoff lichtechte Rotfärbungen mit orangeroter Fluoreszenz.

Analoge Farbstoffe erhält man, wenn man statt des Ausgangsmaterials nach Beispiel 30 äquivalente Mengen der Farbstoffe 1, 4, 31, 34, 41, 46, 55, 64, 68, 73 oder 93 verwendet.

Beispiel 94

In 250 Raumteile o-Dichlorbenzol werden 35 Teile des nach Beispiel 30 erhaltenen Farbstoffs und 27 Teile Diphenylborbromid $(C_6H_5)_2B$ Br eingetragen und auf 150°C erwärmt. Dabei wandeln sich die violetten Kristalle des Farbstoffes 30 in rote Kristalle um, ohne in Lösung zu gehen. Man rührt 2 Stunden bei 150°C, läßt erkalten und saugt ab. Nach dem Waschen (mit Essigsäure und mit Aceton) und Trocknen erhält man 34 Teile der Verbindung der Formel

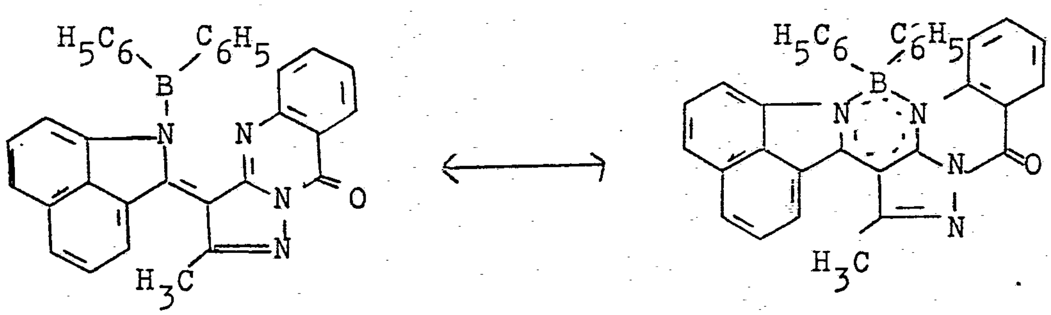

die beim Einarbeiten in thermoplastische Kunststoffe lichtechte Rotfärbungen ergibt.

## Patentansprüche

1. Naphtholactamderivate der allgemeinen Formel I

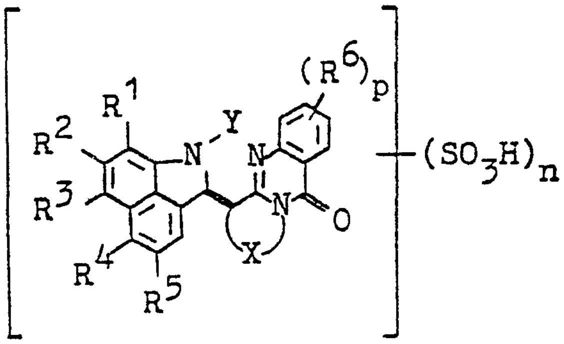

I

in der

n 0, 1, 2 oder 3,

p 0, 1, 2 oder 3

$R^1$ Wasserstoff, Chlor, Brom, Alkyl, Alkoxy, $NO_2$ oder Arylmercapto,

$R^2$ Wasserstoff oder Chlor,

$R^3$ Wasserstoff, Chlor, Brom, Alkyl, Alkoxy, Nitro, Acylamino, Alkylsulfonylamino, Arylsulfonylamino, Alkylmercapto, Arylmercapto, Arylsulfonyl, Alkylsulfonyl, gegebenenfalls N-substituiertes Sulfamid, Alkanoyl, Aroyl, Alkoxycarbonyl, gegebenenfalls substituiertes Carbamoyl oder Cyan,

$R^4$ Wasserstoff, Chlor, Alkoxy, Arylmercapto, Alkylmercapto, Alkoxycarbonyl, gegebenenfalls substituiertes Carbamoyl oder Cyan,

$R^3$ und $R^4$ zusammen einen Rest der Formel

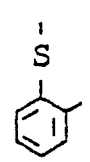

oder

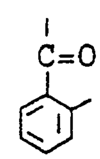

,

$R^5$ Wasserstoff, Chlor oder Alkoxy,

$R^6$ Fluor, Chlor, Brom, Methyl, Trifluormethyl, Nitro, gegebenenfalls substituiertes Carbamoyl oder Sulfamoyl oder Phenylmercapto,

X einen Rest der Formel
-CONH-, -CONR-, -CSNH-, -C=N-, -C=N- oder
SR (am C von -C=N-), $C_1$-$C_8$-Alkyl (am C von -C=N-)

-C=N- und
Aryl (am C)

Y Wasserstoff oder B(T)(T) bedeuten, wobei

T Fluor, Chlor, Brom, gegebenenfalls substituiertes Aryl oder ein Acyloxyrest ist.

2. Verbindungen gemäß Anspruch 1 mit n = 0.

3. Verbindungen gemäß Anspruch 1 der Formel

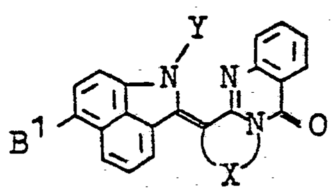

I a

in der
$B^1$ Wasserstoff, Halogen, Aroyl oder Arylmercapto,
X einen Rest der Formel
-CONR- oder -C=N- und
$CH_3$ (am C)
Y Wasserstoff, $BF_2$, $B(Aryl)_2$, B(Aryl)($OCOC_1$- bis $C_4$-Alkyl)
oder B ($OCOC_1$- bis $C_4$-Alkyl$)_2$
bedeuten.

4. Verwendung der Verbindungen gemäß Anspruch 1 zum Färben synthetischer Fasern oder von Kunststoffen.

0025936

Europäisches Patentamt

# EUROPÄISCHER RECHERCHENBERICHT

Nummer der Anmeldung
EP 80 10 538.

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | FR - A - 2 344 603 (BASF)<br>* Ansprüche *<br>-- | |
| A | US - A - 3 793 337 (T. PADMANATHAN)<br>* Ansprüche *<br>---- | |

KLASSIFIKATION DER ANMELDUNG (Int Cl.[3])

C 09 B 57/06
C 07 D 487/04

RECHERCHIERTE SACHGEBIETE (Int Cl.[3])

C 09 B 57/06
C 07 D 487/04

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Grunden angeführtes Dokument
&: Mitglied der gleichen Patentfamilie, ubereinstimmendes Dokument

Der vorliegende Recherchenbericht wurde fur alle Patentanspruche erstellt.

| Recherchenort | Abschlußdatum der Recherche | Prüfer |
|---|---|---|
| Den Haag | 17-11-1980 | GOLLER |